(19) Europäisches Patentamt
European Patent Office
Office européen des brevets

(11) Veröffentlichungsnummer: **0 119 160**
**B1**

# (12) EUROPÄISCHE PATENTSCHRIFT

(45) Veröffentlichungstag der Patentschrift: **05.11.86**

(21) Anmeldenummer: **84810067.3**

(22) Anmeldetag: **06.02.84**

(51) Int. Cl.⁴: **C 07 C 149/42**, C 07 C 149/437, C 07 D 233/32, C 08 K 5/37, C 09 K 15/28, C 09 K 15/30, C 10 M 105/72

## (54) N-substituierte (4-Hydroxyphenylthiomethyl)-amine oder -ureide.

(30) Priorität: **10.02.83 US 465409**

(43) Veröffentlichungstag der Anmeldung: **19.09.84 Patentblatt 84/38**

(45) Bekanntmachung des Hinweises auf die Patenterteilung: **05.11.86 Patentblatt 86/45**

(84) Benannte Vertragsstaaten: **BE DE FR GB IT NL**

(56) Entgegenhaltungen:
**GB-A-1 406 479**

**CHEMICAL ABSTRACTS, Band 86, Nr. 1, 3. Januar 1977, Seite 437, Nr. 5066m, Columbus, Ohio, US, A.I. MEDVEDEV u.a.: "Synthesis and properties of some new derivatives of 3,5-di-tert-butyl-4-hydroxythiophenol"**

(73) Patentinhaber: **CIBA- GEIGY AG, Klybeckstrasse 141, CH- 4002 Basel (CH)**

(72) Erfinder: **Spivack, John D., 1 Blue Jay Street, Spring Valley New York 10977 (US)**
Erfinder: **Pastor, Stephen D., 112 Union Road, Spring Valley New York 10977 (US)**

**EP 0 119 160 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue N-substituierte (4-Hydroxy-phenylthiomethyl)-amine oder -ureide und deren Verwendung als Stabilisatoren für organisches Material.

Organische polymere Verbindungen wie Kunststoffe oder Harze unterliegen thermischem, oxidativem oder strahlungsinduziertem Abbau. In der Technik ist eine grosse Anzahl von Stabilisatoren für eine Vielzahl von Substraten bekannt. Die Wirksamkeit eines Stabilisators hängt unter anderem von der Art des Substrats, in welcbem er eingesetzt wird, und vom jeweiligen Abbaumechanismus ab. Daher ist es im allgemeinen schwierig, für eine konkrete Anwendung den wirksamsten und ökonomischsten Stabilisator anzugeben.

So kann beispielsweise ein Stabilisator, der die Flüchtigkeit einer Verbindung reduziert, dahingehend wirken, dass er einen Bindungsbruch der Substratmoleküle verhindert. Um eine geringe Versprödung oder die Erhaltung der Elastizität eines Polymeren oder eines Elastomeren zu gewährleisten, kann von einem Stabilisator verlangt werden, dass er übermässige Vernetzungsreaktionen und/oder Kettenbruch verhindert. Um die Vergilbung eines Substrats zu unterbinden, muss verhindert werden, dass Reaktionen des Substrats oder des Stabilisators ablaufen, die zu neuen Chromophorem führen. Weiterhin müssen Probleme der Verarbeitungsstabilität und der Substratverträglichkeit beachtet werden.

Primäre (4-Hydroxyphenylthioalkyl)-amine sind aus C.A. 86, 5066 m (1977) als Antioxidantien bekannt, vermögen aber nicht immer den heutigen Forderungen der Technik zu genügen.

Ueberraschenderweise ist jetzt gefunden worden, dass die N-substituierten (4-Hydroxyphenylthiomethyl)-amine und -ureide der vorliegenden Erfindung eine ungewöhnliche Kombination wünschenswerter Eigenschaften besitzen, wodurch diese Stoffe zu besonders wirksamen und nützlichen Stabilisatoren werden. Die erfindungsgemässen Verbindungen erweisen sich als besonders geeignete Stabilisatoren für Polyolefine, Polyester, schlagfestes Polystyrol, ABS, Kautschukarten wie Polybutadien und Styrol-Butadienkautschuk und andere, bei denen der Erhalt der Elastizität und die Verhinderung von Vernetzungsreaktionen, von Verfärbung, von Geruchsbildung und von Ausschwitzen des Stabilisators grundlegende Anforderungen an die Qualität eines Stabilisators sind.

Die Erfindung betrifft demnach Verbindungen der Formel I oder II

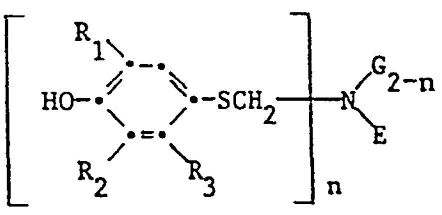

(I)

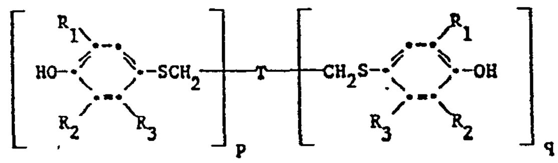

(II)

worin

$R_1$ und $R_2$ unabhängig voneinander $C_1$-$C_{18}$-Alkyl, $C_5$-$C_6$-Cycloalkyl, unsubstituiertes oder durch $C_1$-$C_{12}$-Alkyl substituiertes Phenyl, $C_7$-$C_9$-Aralkyl oder durch $C_1$-$C_{12}$-Alkyl substituiertes Aralkyl sind;

$R_2$ auch Wasserstoff darstellen kann;

$R_3$ Wasserstoff oder Methyl ist,

G und E unabhängig voneinander unsubstituiertes oder durch $C_1$-$C_{12}$-Alkyl oder Phenyl substituiertes Phenyl sind,

G auch Wasserstoff oder $C_1$-$C_{18}$-Alkyl sein kann

n gleich 1 oder 2 ist

T -NH-Phenylen-NH-,

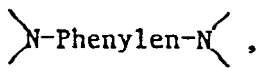 ,

-NH-Phenylen-Q-Phenylen-NH-,

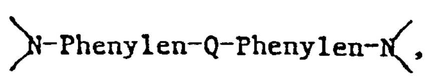 ,

wo Q $C_1$-$C_6$-Alkylen, $C_{2}$-$C_6$-Alkyliden, $-SO_2-$, -SO-, -S-, -S-S-, -O- oder -CO- ist, oder T -NHCONH- oder

T -NHCONH- oder - 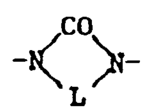 - ist,

ist,

wo L ein grad- oder verzweigtkettiges $C_2$-$C_3$-Alkylen ist und p und q unabhängig voneinander 1 oder 2 sind, wobei die Summe P + q gleich der Valenz von T ist.

$R_1$ und $R_2$ sind als $C_1$-$C_{18}$-Alkyl z.B. Methyl, Ethyl, Isopropyl, n-Butyl, tert.-Butyl, tert.-Amyl, 2-Ethylhexyl, 1,1,3 3-Tetramethyl-butyl, n-Octyl, n-Dodecyl oder n-Octadecyl. $R_1$ und $R_2$ sind bevorzugt verzweigtes $C_4$-$C_8$-Alkyl, wie beispielsweise tert.-Butyl, tert.-Amyl oder 1,1,3,3-Tetramethylbutyl. Besonders bevorzugt sind $R_1$ und $R_2$ tert.-Butyl.

$R_3$ ist bevorzugt Wasserstoff

G und E sind vorzugsweise Phenyl oder durch $C_1$-$C_8$-Alkyl, wie z.B. Methyl, n-Butyl, n-Octyl, bevorzugt Methyl, substituiertes Phenyl.

G ist als $C_1$-$C_{18}$-Alkyl z.B. Methyl, Ethyl, n-Butyl, Octyl, Dodecyl oder Octadecyl.

E ist bevorzugt Phenyl und bei n = 1 ist G ebenfalls bevorzugt Phenyl

In den Definitionen von T stellt Phenylen 1,2-Phenylen, 1,3-Phenylen oder bevorzugt 1,4-Phenylen dar.

Q ist als $C_1$-$C_6$-Alkylen z.B. Methylen, Ethylen, Trimethylen, Hexamethylen. Bevorzugt ist Methylen.

Q ist als $C_2$-$C_6$-Alkyliden z.B. Ethyliden, 2,2-Propyliden oder 1,1-Butyliden.

L ist als $C_2$-$C_3$-Alkylen z.B. Ethylen, Propylen oder Trimethylen. Bevorzugt ist Ethylen.

p und q sind vorzugsweise jeweils 1 oder 2, insbesondere 2.

Die Amine der vorliegenden Erfindung können durch Umsetzung eines aromatischen Amins mit einem Mercaptophenol und Formaldehyd, gegebenenfalls in Gegenwart eines organischen Lösungsmittels wie z.B Methanol, bei Zimmertemperatur hergestellt werden. Das aromatische Amin kann ein Monoamin, wie z.B. Anilin oder Diphenylamin, oder ein Polyamin, wie z.B. p-Phenylendiamin oder 4,4'-Methylendianilin sein. Die Stöchiometrie hängt vom erwünschten Produkt ab.

Als Formaldehyd-Lieferant ist Formalin, eine 37 %ige wässrige Formaldehydlösung besonders geeignet. Geeignet sind aber auch Paraformaldehyd und Trioxan.

Zur Herstellung der Ureide der vorliegenden Erfindung ist es besonders zweckmässig zuerst das entsprechende Hydroxymethylderivat des Ureids herzustellen, z.B. durch Umsetzung von Harnstoff mit Formaldehyd, und anschliessend das Hydroxymethylureid mit dem Mercaptophenol in einem niederen Alkohol, wie z.B. Methanol, in Anwesenheit eines Ueberschusses an wasserfreiem Chlorwasserstoff umzusetzen.

Die Ausgangsprodukte sind im Handel erhältlich oder können nach allgemein bekannten Methoden hergestellt werden. Die Herstellung von Mercaptophenolen ist in E.B. Hotelling et al., J.0rg. Chem. 24, 1598 (1959) beschrieben.

Die als Zwischenprodukte benötigte Mercaptophenole entsprechen der Formel

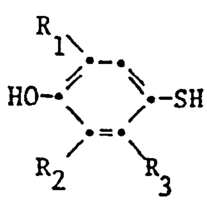

worin $R_1$, $R_2$ und $R_3$ die oben angegebene Bedeutung haben. Bevorzugte Mercaptophenole sind z.B. 2,6-Di-tert.-butyl- und 2-tert.-Butyl-6-methyl-4-mercaptophenol sowie 2,6-Dimethyl-4-mercaptoPhenol und 2-tert.-Butyl-5-methyl-4-mercaptophenol.

Die erfindungsgemässen Verbindungen sind als Stabilisatoren für organisches Material wie Kunststoffe und Schmiermittel gegen thermischem, oxidativem oder strahlungsinduziertem Abbau geeignet, beispielsweise für:

1. Polymere von Mono- und Diolefinen, beispielsweise Polyethylen (das gegebenenfalls vernetzt seim kann), Polypropylen, Polyisobutylen, Polybuten-1, Polynethylpenten-1, Polyisopren oder Polybutadien sowie Polymerisate von Gycloolefinen wie z.B. von Cyclopenten oder Norbornen.

2. Mischungen der unter 1) genannten Polymeren, z.B. Mischungen von Polypropylen mit Polyisobutylen.

3. Copolymere von Mono- und Diolefinen untereinander oder mit anderen Vinylmonomeren, wie z.B. Ethylen-Propylen-Copolymere, Propylen-Buten-1-Gopolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Maleinanhydrid, Styrol-Acrylnitril-Methylacrylat; Mischungen von hoher Schlagzähigkeit aus Styrol-Copolymeren und einem anderen Polymer wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS, MBS, ASA oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von α, β-ungesättigten Säuren und deren Derivaten ableiten, wie Polyacrylate und Polymethacrylate,

Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z.B. Ethylenoxid enthalten.

13. Polyphenyloxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Block-Copolymere mit Polyethern wie z.B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyetherester, die sich von Polyethern mit Hydroxylendgruppen ableiten.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten, oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren wie z.B. PP/EPDM, Polyamid 6/EPDM oder ABS, PVC/EVA, PVC/ABS, PVC/MBS, PC/ABS, PBTP/ABS.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellithate), sowie Abmischungen synthetischer Ester mit Mineralölen in beliebigen Gewichtsverhältnissen, wie sie z.B. als Spinnpräparationen Anwendung finden, sowie deren wässrigen Emulsionen.

29. Wässrige Emulsionen natürlicher oder synthetischer Kautschuke, wie z.B. Naturkautschuk-Latex oder Latices von carboxylierten Styrol-Butadien-Copolymeren.

Die erfindungsgemässen Verbindungen eignen sich insbesondere zum Stabilisieren folgender Polymere: Polyolefine, homo- oder copolymere, wie z.B. Polyethylen, Polypropylen, Polyisobutylen, Poly-(buten-1), Poly-(penten-1), Poly-(3-methylbuten-1), Poly-(4-methylpenten-1), verschiedene Ethylen-Propylen-Copolymere wie EPM, EPDM und dergleichen; Polystyrol sowie Copolymere und Terpolymere von Styrol einschliesslich schlagfestes Polystyrol, ABS, SBR, Polyisopren, Polybutadien sowie natürlicher Kautschuk; Polyester, wie z.B. Polyethylenterephthalat und Polybutylen-terephthalat, und deren Copolymere.

Die Verbindungen der vorliegenden Erfindung eignen sich insbesondere auch zur Stabilisierung mineralischer und synthetischer Schmieröle und -fette.

Die Erfindung betrifft weiterhin Stoffzusammensetzungen enthaltend ein organisches Material, welches thermischem, oxidativem oder strahlungsinduziertem Abbau unterworfen ist und mindestens eine der oben beschriebenen Verbindungen der Formel I oder II.

Die erfindungsgemässen Stabilisatoren werden in Mengen von 0,01 bis 5 Gew.-%, bezogen auf

die gesamte Stoffzusammensetzung in das zu stabilisierende Material eingearbeitet. Je nach Verwendung und Substrat wird die Stabilisatormenge variiert. Bevorzugt verwendet man 0,05 bis 2, insbesondere aber 0,1 bis 1 Gew.-% des Stabilisators.

Die Einarbeitung der Stabilisatorsubstanzen in die organischen Polymeren erfolgt nach bekannten Verfahren. Die Zudosierung kann während jeder Verarbeitungsstufe vor der Formgebung erfolgen. So kann der Stabilisator mit den pulverförmigen Polymeren gemischt werden, oder eine Emulsion oder eine Suspension des Stabilisators kann mit einer Lösung, Suspension oder Emulsion des Polymeren vermischt werden.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Die stabilisierten Polymerzusammensetzungen können gegebenenfalls noch weitere Zusätze enthalten, wie zum Beispiel:

**1. Antioxidantien**

**1.1. Alkylierte Monophenole**

2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

**1.2. Alkylierte Hydrochinone**

2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

**1.3. Hydroxylierte Thiodiphenylether**

2,2'-Thio-bis-(6-tert.butyl-4-methylphenol).
2,2'-Thio-bis-(4-octylphenol)
4,4'-Thio-bis-(6-tert.butyl-3-methylphenol)
4,4'-Thio-bis-(6-tert.butyl-2-methylphenol)

**1.4. Alkyliden-Bisphenole**

2,2,-Methylen-bis-(6-tert.butyl-4-methylphenol)
2,2,-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methlyen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphemol
1,1,3-Tris-(5-tert.butyl-4-hydroxy-2-methylphenyl)-butan
1,1-Bis-(5-tert.butyl-4-hydroxy-2-methylphenyl)-3-n-dodecylmercapto-butan
Ethylenglycol-bis-[3,3-bis-(3'-tert.butyl-4'-hydroxyphenyl)-butyrat]
Di-(3-tert.butyl-4=hydroxy-5-methylphenyl)-dicyclopentadien
Di-[2-(3'-tert.butyl-2'-hydroxy-5'-methylbenzyl)-6-tert.butyl-4-methyl-phenyl]-terephthalat.

**1.5 Benzylverbindungen**

1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz.

**1.6. Acylaminophenole**

4-Hydroxy-laurinsäureanilid
4-Hydroxy-stearinsäureanilid
2,4-Bis-octylmercapto-6-(3,5-di-tert.butyl-4-hydroxyanilino)s-triazin
N-(3,5-di-tert.butyl-4-hydroxyphenyl)-carbaminsäureoctylester.

**1.7. Ester der α-(3,5-Di-tert.butyl-4-hydroxyphenyl)-propionsäure**

mit ein- oder mehrwertigen Alkoholen, wie z.B. mit
Methanol Diethylenglycol
Octadecanol Triethylenglycol
1,6-Hexandiol Pentaerythrit
Neopentylglycol Tris-hydroxyethyl-isocyanurat
Thiodiethylenglycol Di-hydroxyethyloxalsäurediamid

**1.8. Ester der β-(5-tert.butyl-4-hydroxy-3-methylphenyl)-eropionsäure**

mit ein- oder mehrwertigen Alkoholen, wie z.B. mit
Methanol Diethylenglycol
Octadecanol Triethylenglycol
1,6-Hexandiol Pentaerythrit
Neopentylglycol Tris-hydroxyethyl-isocyanurat
Thiodiethylenglycol Di-hydroxyethyloxalsäurediamid

**1.9. Amide der β-(3,5-Di-tert.butyl-4-hydroxyphenyl)-propionsäure**

wie z.B.
N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin

**2. UV-Absorber und Lichtschutzmittel**

**2.1. 2-(2'-Hydroxyphenyl)-benztriazole**

wie z.B. das 5'-Methyl-, 3',5'-Di-tert.butyl-, 5'-Tert.butyl-, 5'-(1,1,3,3-Tetramethyl-butyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

**2.2. 2-Hydroxybenzophenone**

wie z.B. das 4-Hydroxy-, 4-Methoxy-, 4-Octoxy-, 4-Decyloxy- 4-Dodecyloxy- 4-Benzyloxy-, 4,2',4'-Trihydroxy-, 2'-Hydroxy-4,4'-dimethoxy-Derivat.

**2.3. Ester von gegebenenfalls substituierten Benzoesäuren**

wie z.B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

**2.4. Acrylate**

wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin,

**2.5. Nickelverbindungen**

wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triethanolamin oder N-Cyclohexyl-diethanolamin, Nickeldibutyldithiocarbamat, Nickelsalze von 4-Hydroxy-3,5-di-tert.butylbenzyl-phosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyra-zols, gegebenenfalls mit zusätzlichen Liganden.

**2.6 Sterisch gehinderte Amine**

wie z.B. Bis-(2,2,6,6-tetramethyl-piperidyl)-sebacat Bis-(1,2,2,6,6-pentamethylpiperidyl)-sebacat n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-Tetramethyl-4-hydroxypiperidin und Bernsteinsäure, Kondensationsprodukt aus N,N'-(2,2,6,6-Tetramethylpiperidyl)-hexamethylendiamin und 4-tert.octylamino-2,6-dichlor-1,3,5-s-triazin,
Tris-(2,2,6,6-tetramethylpiperidyl)-nitrilotriacetat,
Tetrakis-(2,2,6,6-tetramethyl-4-piperidyl)-1,2,3,4-butantetracarbon-säure,
1,1'-(1,2-ethandiyl)-bis-(3,3,5,5-tetramethyl-piperazinon).

**2.7. Oxalsäurediamide**

wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxamilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.bu-tyl-oxanilid, Gemische von ortho-und para-Methoxy- sowie von o- und p-Ethoxy-di-substituierte Oxaniliden.

**3. Metalldesaktivatoren**

wie z.B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzylidenoxalsäuredihydrazid.

**4. Phosphite und Phosphonite**

wie z.B. Triphenylphosphit, Diphenylalkylphosphite, Phenyldialkylphosphite, Tri-(nonylphenyl)-phosphit, Trilaurylphosphit, Trioctadecylphosphit, Distearylpentaerythritdiphosphit, Tris-(2,4-di-tert.butylphenyl)-phosphit, Diisodecylpentaerythritdiphosphit, Di-(2,4-di-tert.butylphenyl)-pentaerythritdiphosphit, Tristearyl-sorbit-triphosphit, Tetrakis-(2,4-di-tert.bu-tylphenyl)-4,4'-biphenylendiphosphonit.

**5. Peroxidzerstörende Verbindungen**

wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

**6. Polyamidstabilisatoren**

wie z.B. Kupfersalze in Kombination mit Jodiden und/oder Phosphorverbindungen und Salze des zweiwertigen Mangans.

**7. Basische Co-Stabilisatoren**

wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

**8. Nukleierungsmittel**

wie z.B. 4-tert.Butylbenzoesäure, Adipinsäure, Diphenylessigsäure.

**9. Füllstoffe und Verstärkungsmittel**

wie z.B Calciumcarbonat, Silikate, Glasfasern, Asbest, Talk, Kaolin, Glimmer, Bariumsulfat, Metalloxide und -hydroxide, Russ, Graphit.

**10. Sonstige Zusätze**

wie z.B. Weichmacher, Gleitmittel, Emulgatoren, Pigmente, Optische Aufheller, Flammschutzmittel, Antistatika, Treibmittel.

Die erfindungsgemässen Verbindungen der Formel I können als alleiniger Stabilisator verwendet werden und wirken dann hauptsächlich als Antioxidans oder als Lichtschutzmittel oder sie wirken sowohl als Antioxidans und als Lichtschutzmittel.

Die folgenden Beispiele erläutern die Erfindung ohne sie zu begrenzen.

**Beispiel 1:**

N-(3,5-Di-tert.-butyl-4-hydroxyphenylthiomethyl)-N,Ndiphenylamin.

Eine Mischung aus 8,46 g Diphenylamin, 11,92 g 2,6-Di-tert.-butyl-4-mercaptophenol, 4,06 g 37 %iges wässriges Formaldehyd und 35 ml Methanol wird in einem 100 ml Kolben unter Stickstoff über Nacht gerührt. Das Lösungsmittel wird anschliessend im Vakuum abdestilliert und der Rückstand aus Petrolether umkristallisiert. Man erhält 12,02 g (57 % d.Th.) einer weissen kristallinen Substanz mit Smp. 71-75° C.

| Analyse: | C | H | N |
|---|---|---|---|
| berechnet | 77,3 | 7,9 | 3,3 |
| gefunden | 77,4 | 8,0 | 3,3 |

**Beispiel 2**

N-(3,5-Di-tert.-but 1-4-hydroxyphenylthiomethyl)-N,N-bis-(p-1,1,3,3-tetramethylbutylphenyl)-amin.

Das Verfahren gemäss Beispiel 1 wird wiederholt mit der einzigen Ausnahme, dass anstelle von Diphenylamin die äquivalente Menge Bis-(p-1,1,3,3-tetramethylbutylphenyl)-amin eingesetzt wird. Man erhält die obengenannte Verbindung mit Smp. 105-108° C.

| Analyse: | C | H | N |
|---|---|---|---|
| berechnet | 67,5 | 8,6 | 4,8 |
| gefunden | 67,3 | 8,7 | 4,8 |

**Beispiel 3**

N,N'-Bis-(3,5-di-tert.-butyl-4-hydroxyphenylthiomethyl)urea.

11,92 g 2,6-Di-tert.-butyl-4-mercaptophenol und 3,00 g 1,3-Bis-hydroxymethyl)-harnstoff, in 100 ml Methanol gelöst,werden in einem 500 ml Kolben unter Stickstoff mit einem Ueberschuss an wasserfreiem gasförmigem Chlorwasserstoff behandelt. Das Reaktionsgemisch wird über Nacht gerührt, die Festsubstanz anschliessend abfiltriert, mit Methanol gewaschen und getrocknet. Man erhält 9,4 g (67 % d.Th.) einer weissen festen Substanz mit Smp. 213-216° C.

| Analyse: | C | H | N |
|---|---|---|---|
| berechnet | 66,4 | 8,6 | 5,0 |
| gefunden | 66,6 | 8,4 | 5,1 |

**Beispiel 4**

1,3-Bis-(3,5-di-tert.-butyl-4-hydroxyphenylthiomethyl)-2-imidazolidon

Eine Lösung von 3,65 g 1,3-Bis-(hydroxymethyl)-2-imidazolidon und 11,92 g 2,6-Di-tert.-butyl-4-mercaptophenol in 100 ml Methanol wird in einem 300 ml Kolben unter Stickstoff mit einem Ueberschuss an wasserfreiem Chlorwasserstoff behandelt. Das Reaktionsgemisch wird abgekühlt und der Rückstand aus Methanol umkristallisiert. Man erhält 11,43 g eines weissen festen Stoffs mit Smp. 173-176° C.

**Beispiel 5**

N,N-Bis-(3,5-di-tert.-butyl-4-hydroxyphenylthiomethyl)-N-phenylamin

Eine Mischung aus 4,66 g Anilin, 8,11 g 37 %iges wässeriges Formaldehyd, 23,84 g 2,6-Di-tert.-butyl-4-mercaptophenol und 35 ml Methanol wird in einem 100 ml Kolben unter Stickstoff über Nacht gerührt. Die Festsubstanz wird dann abfiltriert und aus Methanol umkristallisiert. Man erhält 23,38 g (83 % d.Th.) eines weissen festen Stoffs mit Smp. 119-121° C.

| Analyse: | C | H | N |
|---|---|---|---|
| berechnet | 72,8 | 8,7 | 2,4 |
| gefunden | 72,9 | 8,9 | 2,5. |

**Beispiel 6**

N,N,N',N'-Tetrakis-(3,5-di-tert.-butyl-4-hydroxyphenylthiomethyl)-p-phenylendiamin.

Eine Mischung aus 2,70 g p-phenylendiamin, 8,12 g 37 %iges wässriges Formaldehyd, 23,84 g 2,6-Di-tert.-butyl-4-mercaptophenyl und 35 ml Methanol wird in einem 100 ml Kolben unter Stickstoff über Nacht gerührt. Die Festsubstanz wird dann abfiltriert und aus einem Heptan/Toluol-Gemisch umkristallisiert. Man erhält 17,92 g (65 % d.Th.) eines weissen festen Stoffs mit Smp. 150-160° C.

| Analyse: | C | H | N |
|---|---|---|---|
| berechnet | 71,4 | 8,7 | 2,5 |
| gefunden | 71,5 | 8,7 | 2,7 |

**Beispiel 7**

In diesem Beispiel wird die stabilisierende Wirkung der erfindungsgemässen Verbindungen in schlagfestem Polystyrol (IPS) demonstriert.

Herstellung der Proben: Man stellt eine Lösung von 8 Gew.-% Polybutadien-Kautschuk (Firestone DIENE 55) in monomerem Styrol her, indem man beide Komponenten in einer Quetschmühle mischt. Gleichzeitig wird in diesem Arbeitsgang der jeweils gewünschte Anteil des zu testenden Stabilisators zu der Lösung gegeben. Schliesslich fügt man noch 500 ppm Zn-Stearat hinzu, um später die polymerisierte Probe leicht aus dem Reaktionsgefäss ablösen zu können. Die Polymerisation wird in einem Gefäss durchgeführt, dass mit einem Wendelrührer ausgerüstet ist. Da die meisten IPS Blockpolymerisationen thermisch initiiert werden, wird in der vorliegenden Laboratoriumsversion kein Starter zugesetzt. Die Reaktion wird unter Stickstoff und unter Erwärmen durchgeführt. Der Reaktor wird innerhalb einer halben Stunde auf 121° C erwärmt, und diese Temperatur wird aufrecht erhalten, bis zwischen 30 und 35 % des Monomeren umgesetzt sind (Zeitdauer ca.21/2 Stunden). Während der Polymerisation wird heftig gerührt, und die Rührgeschwindigkeit wird so eingestellt, dass Polymerpartikel von 2 bis 4 µm Grösse entstehen. Anschliessend werden die Reaktionsgefässe dem Reaktor entnommen, im Stickstoffstrom geöffnet und in ein Wirkbelschichtsandbad gestellt, um die Polymerisation zu vervollständigen. Die Gefässe werden im Sandbad folgendermassen erhitzt:

eine Stunde bei 100° C um die Anfangstemperatur vorzugeben, eine weitere Stunde, um 140° C zu erreichen und anschliessend weitere 8 Stunden, wobei die Temperatur jede Stunde um 10° C erhöht wird, bis schliesslich ein Maximum von 220° C erreicht ist. Nach dem Abkühlen werden die Polymerisationsgefässe zerbrochen und das Glas entfernt. Ein einziger Polymerblock wiegt im Mittel etwas über 600 g. Der Block wird in einem Vakuumofen bei 200° C für 45 Minuten bei einem Druck von 1,33 mbar belassen, um flüchtige Bestandteile zu entfernen. Nach dieser Behandlung wird der Block sofort in einer heizbaren hydraulischen Presse bei 205° C zwischen zwei Aluminiumfolien zu einem dicken Stab gepresst (drei Minuten Erhitzen, fünf Minuten ungeheizt). Der Stab wird mittels einer Handsäge zerkleinert und anschliessend granuliert. Alle Polymermischungen werden bei 205° C extrudiert und dann zerkleinert. Die Pellets werden unter Druck bei 205° C in dehnbare 3,175 mm dicke Streifen verformt.

Die Probestreifen werden dann bei 150° C in einem Umluftofen gealtert, wobei sie auf Glasplatten, die sich wiederum auf rotierenden Trägern befinden, aufgebracht sind. Eine weitere Charge von Probestreifen wird auf dieselbe Weise bei 80° C im Ofen gealtert. Nach bestimmten Zeitintervallen wird der Yellowness Index der Proben gemäss ASTM D-1925-63T sowie die Dehnbarkeit gemäss ASTM D 638 (Instron Tensile Testing Apparatus, Instron Engineering Corporation, Massachusetts; Ziehgeschwindigkeit: 5 mm/Minute) bestimmt.

In der folgenden Tabelle 1a sind die Ergebnisse der Dehnbarkeitsmessungen und der Messungen des Yellowness-Index von bei 80° C ofengealterten Proben angegeben, während Tabelle 1b Messwerte von bei 150° C ofengealterten Proben enthält.

**Tabelle Ia:** Dehnbarkeitsmessungen und Yellowness Index von bei 80° C ofengealterten Proben mit und ohne Stabilisator

| Additiv | Stabilisator-menge (Gew.-%) | Dehnbarkeit der Proben (%) und Dauer der Hitzeeinwirkung (h) | | | | |
|---|---|---|---|---|---|---|
| | | 0 h | 300 h | 600 h | 900 h | 1 200 h |
| Produkt von Beispiel 1 | 0,1 | 40 | 24 | 17 | 12 | 9 |
| — | — | 33 | 9 | 3 | 3 | 3 |

| Additiv | Stabilisator-menge (Gew.-%) | Yellonness Index und Dauer der Hitzebelastung (h) | | | | |
|---|---|---|---|---|---|---|
| | | 0 h | 300 h | 600 h | 900 h | 1 200 h |
| Produkt von Beispiel 1 | 0,1 | −3 | 1 | 11 | 25 | 30 |
| — | — | 7 | 14 | 45 | 59 | — |

**Tabelle Ib:** Dehnbarkeitsmessungen und Yellowness Index von bei 150°C ofengealterten Proben mit und ohne Stabilisator

| Additiv | Stabilisatormenge (Gew.-%) | Dehnbarkeit der Probe (%) und Dauer der Hitzebelastung (h) | | | | |
|---|---|---|---|---|---|---|
| | | 0 h | ½ h | 1 h | 1½ h | 2 h |
| Produkt von Beispiel 1 | 0,1 | 36 | 30 | 13 | 13 | 10 |
| — | — | 33 | 7 | 7 | 3 | 3 |

| Additiv | Stabilisatormenge (Gew.-%) | Yellowness Index und Dauer der Hitzebelastung (h) | | | | |
|---|---|---|---|---|---|---|
| | | 0 h | ½ h | 1 h | 1½ h | 2 h |
| Produkt von Beispiel 1 | 0,1 | − 3 | − 3 | 3 | 4 | 7 |
| — | — | 7 | 18 | 30 | 38 | 43 |

**Beispiel 8**

Unstabilisiertes Polypropylenpulver (Hercules Profax 6501) wird mit der in Tabelle 2 angegebenen Menge des Additivs gemischt. Diese Mischungen werden anschliessend bei 182°C 5 Minuten lang auf einem Mischwalzwerk plastifiziert. Anschliessend wird die stabilisierte Polypropylenfolie vom Walzwerk abgelöst und abkühlen gelassen. Die Folie wird in Stücke geschnitten und auf einer hydraulischenPresse im Oel-Oxidationstest gemäss ASTM D 943-81 bei 220°C und 12 bar zu einer 0,635 mm dicken Probe verformt. Diese Probe wird in einer Fluoreszenzsonnenlicht/ Schwarzlichtkammer bis zur Zersetzung belichtet. Als Zersetzungszeitpunkt wird diejenige Anzahl von Stunden gewertet, bei der die IR-spektroskopisch gemessene dekadische Extinktion der Carbonylbande 50 % des Anfangswertes erreicht. Die Messdaten sind in der folgenden Tabelle 2 dargestellt:

**Tabelle 2**

| Additiv (Produkt von Beispiel) | Stabilisatormenge (Gew.-%) | Zahl der Stunden bis zur Zersetzung der bestrahlten Proben |
|---|---|---|
| 1 | 0,2 | 400 |
| 3 | 0,2 | 320 |
| 4 | 0,2 | 360 |
| 5 | 0,2 | 460 |
| — | — | 200—300 |

**Beispiel 9**

Oel-Oxidationstest

Eine Oelprobe von 300 ml Turbinenöl (Exxon LO 5084 ®) enthaltend 0,25 Gew.-% der Verbindung von Beispiel 1 und 60 ml destilliertes Wasser wird geprüft. Bestimmt wird die Zeit (in Stunden) bis zur Zersetzung, d.h. bis zur Erreichung einer Säurezahl von 2,0, der Oelprobe.

Die Resultate sind in der nachstehenden Tabelle 3 aufgeführt:

**Tabelle 3**

| Additiv (Produkt von Beispiel) | Stabilisatormenge (Gew.-%) | Zahl der Stunden bis zur Erreichung von Säurezahl 2,0 |
|---|---|---|
| 1 | 0,25 | 3 700 |
| — | — | 140 |

**Beispiel 10**

Oel-Oxidationstest

Eine Oelprobe von 160 g Flugzeugturbinenöl "Royal Mixed Polyol Ester Aircraft Base Oil" der Firma Royal Lubricant Co. enthaltend 1,0 Gew.-% der Verbindung von Beispiel 1 wird in einem Rohr aufgenommen. Durch die auf 202,2° C erhitzte Oelmischung wird mit einer Geschwindigkeitsrate von 3 Liter/Stunde trockene Luft geleitet.

Der Test wird beim Erreichen des ersten der beiden folgenden Endpunkten abgebrochen: entweder nach 48 Stunden, wobei am Schluss die Säurezahl gemessen wird, oder wenn die Säurezahl 4,0 erreicht ist.

Die mit der Verbindung von Beispiel 1 stabilisierte Oelprobe weist nach 48 Stunden immer noch eine Säurezahl von 1,1, während eine unstabilisierte Oelprobe bereits nach weniger als 24 Stunden die Säurezahl 4,0 erreicht.

**Patentansprüche**

1. Verbindungen der Formeln I oder II

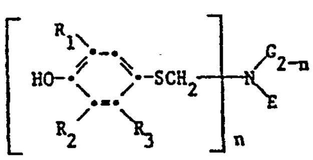
(I)

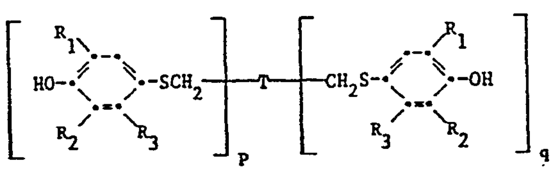
(II)

worin

$R_1$ und $R_2$ unabhängig voneinander $C_1$-$C_{18}$-Alkyl, $C_5$-$C_6$-Cycloalkyl, unsubstituiertes oder durch $C_1$-$C_{12}$-Alkyl substituiertes Phenyl, $C_7$-$C_9$-Aralkyl oder durch $C_1$-$C_{12}$-Alkyl substituiertes Aralkyl sind;

$R_2$ auch Wasserstoff darstellen kann;

$R_3$ Wasserstoff oder Methyl ist,

G und E unabhängig voneinander unsubstituiertes oder durch $C_1$-$C_{12}$-Alkyl oder Phenyl substituiertes Phenyl sind,

G auch Wasserstoff oder $C_1$-$C_{18}$-Alkyl sein kann

n gleich 1 oder 2 ist

T -NH-Phenylen-NH-,

>N-Phenylen-N< ,

-NH-Pbenylen-Q-Phenylen-NH-,

>N-Phenylen-Q-Phenylen-N<,

wo Q $C_1$-$C_6$-Alkylen, $C_2$-$C_6$-Alkyliden, -$SO_2$-, -SO-, -S-, -S-S-, -O- oder -CO- ist, oder

T -NHCONH- oder

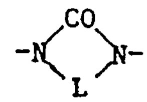

ist,

wo L ein grad- oder verzweigtkettiges $C_2$-$C_3$-Alkylen ist und p und q unabhängig voneinander 1 oder 2 sind, wobei die Summe p + q gleich der Valenz von T ist.

2. Verbindungen der Formel I oder II gemäss Anspruch 1, worin $R_1$ und $R_2$ verzweigtes $C_4$-$C_8$-Alkyl bedeuten.

3. Verbindungen der Formel I oder II gemäss Anspruch 2, worin $R_1$ und $R_2$ tert.-Butyl bedeuten.

4. Verbindungen der Formel I oder II gemäss Anspruch 1, worin $R_3$ Wasserstoff bedeutet.

5. Verbindungen der Formel I gemäss Anspruch 1, worin E und G Phenyl und n = 1 sind.

6. Verbindungen der Formel I gemäss Anspruch 1, worin n 1 oder 2 ist und E Phenyl bedeutet.

7. Verbindungen der Formel II gemäss Anspruch 1, worin in den Definitionen von T, Phenylen 1,4-Phenylen bedeutet.

8. Verbindungen der Formel II gemäss Anspruch 1, worin T -NH-Phenylen-Q-Phenylen-NH- oder

>N-Phenylen-Q-Phenylen-N<

und Q Methylen bedeuten.

9. Verbindungen der Formel II gemäss Anspruch 1, worin T eine Gruppe der Formel

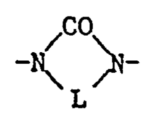

und L Ethylen bedeuten.

10. Verbindungen der Formel II gemäss Anspruch 2, worin p und q jeweils = 1 oder 2 sind.

11. Verbindungen der Formel 11 gemäss Anspruch 10, worin p und q = 2 sind.

12. Stoffzusammensetzung enthaltend ein organisches Material, welches thermischem, oxidativem oder strahlungsinduziertem Abbau unterworfen ist und mindestens eine Verbindung der Formel I oder II gemäss Anspruch 1.

13. Stoffzusammensetzung gemäss Anspruch 12, dadurch gekennzeichnet, dass das organische Material ein synthetisches Polymer ist.

14. Stoffzusammensetzung gemäss Anspruch 13, dadurch gekennzeichnet, dass das Polymere ein polyolefinisches Homo- oder Copolymere ist.

15. Stoffzusammensetzung gemäss Anspruch 13, dadurch gekennzeichnet, dass das Polymere ein Homopolymere, Copolymere oder Terpolymere von Styrol ist.

16. Stoffzusammensetzung gemäss Anspruch 12, dadurch gekennzeichnet, dass das organische Material ein mineralisches oder synthetisches Schmieröl ist.

**Claims**

1. A compound of formula I or II

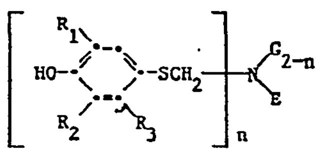

(I)

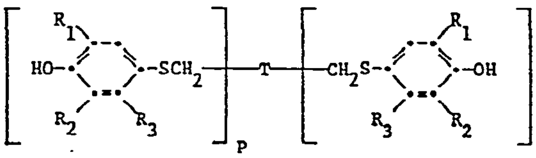

(II)

wherein $R_1$ and $R_2$ are independently alkyl of 1 to 18 carbon atoms, cycloalkyl of 5 to 6 carbon atoms, phenyl, phenyl substituted by alkyl of 1 to 12 carbon atoms, or are aralkyl of 7 to 9 carbon atoms or aralkyl substituted by alkyl of 1 to 12 carbon atoms; $R_2$ may also represent hydrohydrogen;

$R_3$ is hydrogen or methyl;

G and E are independently phenyl or phenyl substituted by alkyl of 1 to 12 carbon atoms or by phenyl;

G may also be hydrogen or alkyl of 1 to 18 carbon atoms;

n is 1 or 2,

T is -NH-phenylene-NH-,

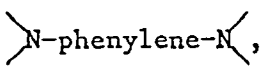,

-NH-phenylene-Q-phenyle-ne-NH-,

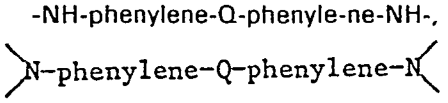

where Q is alkylene of 1 to 6 carbon atoms, alkylidene of 2 to 6 carbon atoms, $-SO_2-$, -SO-, -S-, -S-S-, -O- or -CO-, or

T is -NHCONH- or

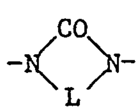

where L is a straight or branched chain alkylene of 2 to 3 carbon atoms, and

p and q are independently 1 or 2, with the sum of p plus q being equal to the valence of T.

2. A compound of formula I or II according to claim I, wherein $R_1$ and $R_2$ are branched alkyl of 4 to 8 carbon atoms.

3. A compound of formula I or II according to claim 2, wherein $R_1$ and $R_2$ are each tert-butyl.

4. A compound of formula 1 or II according to claim 1, wherein $R_3$ is hydrogen.

5. A compound of formula I according to claim 1, wherein E and G are each phenyl and n is 1.

6. A compound of formula I according to claim 1, wherein n is 1 or 2 and E is phenyl.

7. A compound of formula II according to claim 1, wherein in the definitions of T, phenylene is 1,4-phenylene.

8. A compound of formula II according to claim 1, wherein T is -NHphenylene-Q-phenylene-NH- or

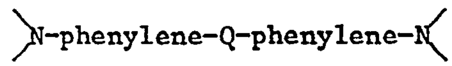

and Q is methylene.

9. A compound of formula II according to claim 1, wherein T is a group of the formula

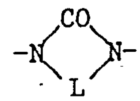

and L is ethylene.

10. A compound of formula II according to claim 2, wherein p and q are each 1 or 2.

11. A compound of formula II according to claim 10, wherein p and q are each 2.

12. A composition comprising an organic material subject to thermal, oxidative or radiation-induced degradation, and at least one compound of formula I or II according to claim 1.

13. A composition according to claim 12, wherein the organic material is a synthetic polymer.

14. A composition according to claim 13, wherein the polymer is a polyolefin homopolymer or copolymer.

15. A composition according to claim 13, wherein the polymer is a styrene homopolymer, copolymer or terpolymer.

16. A composition according to claim 12, wherein the organic material is a mineral or a synthetic lubricating oil.

**Revendications**

1. Composés répondant à l'une des formules I et II:

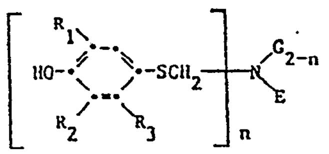

(I)

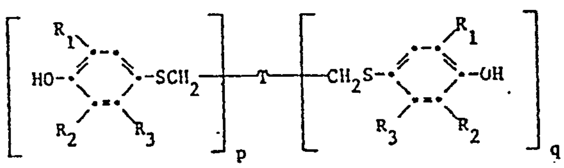

(II)

dans lesquelles:

$R_1$ et $R_2$ représentent chacun, indépendamment l'un de l'autre, un alkyle en $C_1$-$C_{18}$, un cycloalkyle en $C_5$ ou $C_6$, un phényle non substitué ou porteur d'un alkyle en $C_1$-$C_{12}$, un aralkyle en $C_7$-$C_9$ ou un aralkyle porteur d'un alkyle en $C_1$-$C_{12}$, $R_2$ pouvant également représenter l'hydrogène,

$R_3$ représente l'hydrogène ou un méthyle,

G et E représentent chacun, indépendamment l'un de l'autre, un phényle non substitué ou porteur d'un alkyle en $C_1$-$C_{12}$ ou d'un phényle, G pouvant également représenter l'hydrogène ou un alkyle en $C_1$-$C_{18}$,

n est égal à 1 ou à 2,

T représente un radical -NH-phénylène-NH-,

>N-phénylène-N<

-NH-phénylène-Q-phénylène-NH- ou

>N-phénylène-Q-phénylène-N<,

où Q représente un alkylène en $C_1$-$C_6$, un alkylidène en $C_2$-$C_6$, $-SO_2-$,-SO-,-S-, -S-S-, -O- ou -CO-, ou encore T représente un radical -NHCONH- ou un radical

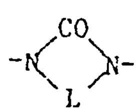

dans lequel L représente un alkylène en $C_2$ ou $C_3$ linéaire ou ramifié, et

p et q représentent chacun, indépendamment l'un de l'autre, le nombre 1 ou le nombre 2, la somme (p+q) étant égale à la valence de T.

2. Composés de formule I ou II selon la revendication 1, dans lesquels $R_1$ et $R_2$ représentent chacun un radical alkyle ramifié en $C_4$-$C_8$.

3. Composés de formule I ou II selon la revendication 2, dans lesquels $R_1$ et $R_2$ représentent chacun un radical tert-butyle.

4. Composés de formule I ou II selon la revendication 1, dans lesquels $R_3$ représente l'hydrogène.

5. Composés de formule I selon la revendication 1, dans lesquels E et G représentent un radical phényle et n est égal à 1.

6. Composés de formule I selon la revendication 1, dans lesquels n est égal à 1 ou à 2 et E représente un radical phényle.

7. Composés de formule II selon la revendication 1, dans lesquels la partie phénylène, dans les définitions de T, est un radical phénylène-1,4.

8. Composés de formule II selon la revendication 1, dans lesquels T représente un radical -NH-phénylène-Qphénylène-NH- ou

>N-phénylène-Q-phénylène-N<

et Q représente un radical méthylène.

9. Composés de formule II selon la revendication 1, dans lesquels T représente un radical répondant à la formule:

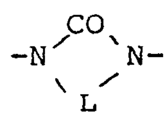

dont le symbole L désigne un radical éthylène.

10. Composés de formule II selon la revendication 2, dans lesquels p et q sont égaux chacun à 1 ou à 2.

11. Composés de formule II selon la revendication 18, dans lesquels p et q sont égaux chacun à 2.

12. Composition contenant une matière organique susceptible de subir une dégradation sous l'action de la chaleur, de l'oxydation ou d'un rayonnement, composition qui contient au moins un composé de formule I ou de formule II selon la revendication 1.

13. Composition selon la revendication 12 caractérisée en ce que la matière organique est un polymère synthétique.

14. Composition selon la revendication 13 caractérisée en ce que le polymère est un homopolymère ou un copolymère polyoléfinique.

15. Composition selon la revendication 13 caractérisée en ce que le polymère est un homopolymère, un copolymère ou un terpolymère du styrène.

16. Composition selon la revendication 12 caractérisée en ce que la matière organique est une huile lubrifiante minérale ou synthétique.